# EUROPÄISCHE PATENTANMELDUNG

(11) **EP 1 382 314 A2**
(43) Veröffentlichungstag der Anmeldung: **21.01.2004**
(21) Anmeldenummer: 03400030.7
(22) Anmeldetag: 04.06.2003
(51) Int. Cl.: A61F 2/30, A61F 2/38

(54) **Künstliches Gelenk**

(30) Priorität: 11.07.2002 DE 10231538
(71) Anmelder: HJS Gelenk System GmbH, 80925 München (DE)
(72) Erfinder: Kubein-Meesenburg, Dietmar, Prof. Dr., 37075 Göttingen (DE); Nägerl, Hans, Prof. Dr., 37130 Gleichen (DE); Adam, Peter, Prof. Dr., 85221 Dachau (DE); Theusner, Joachim, Dr., 80539 München (DE)
(74) Vertreter: Scheffler, Jörg

(57) **Zusammenfassung**

Die Erfindung betrifft ein insbesondere als Endoprothese für das menschliche Kniegelenk bestimmtes künstliches Gelenk (1), umfassend ein erstes, durch einen ersten Gelenkkopf (3) und eine erste Gelenkpfanne (4) gebildetes Gelenkkompartiment (2) und ein zweites durch einen zweiten Gelenkkopf (6) und eine zweite Gelenkpfanne (7) gebildetes Gelenkkompartiment (5), wobei Kontaktflächen (C) der jeweiligen Gelenkkompartimente (2, 5) in der Hauptfunktionsebene einen Versatz aufweisen. Um die Eigenschaften des künstlichen Gelenkes (1) weiter zu verbessern, wird erfindungsgemäß vorgeschlagen, die Kontaktflächen (C) der beiden Gelenkkompartimente (2, 5) in Abhängigkeit des Flexionswinkels derart geneigt anzuordnen, dass die Flächennormalen (8, 9) der Kontaktflächen (C) bei jedem Flexionswinkel einen gemeinsamen Schnittpunkt aufweisen. Durch diese Anordnung der Kontaktflächen (C) wird eine Selbststabilisierung des Gelenkes (1) sowohl bei der Einleitung einer Rotationsbewegung bzw. Torsion, als auch bei Einleitung seitlicher Kräfte erreicht.

## Beschreibung

Die Erfindung betrifft ein insbesondere als Endoprothese für das menschliche Kniegelenk bestimmtes künstliches Gelenk, umfassend ein erstes, durch einen ersten Gelenkkopf und eine erste Gelenkpfanne gebildetes Gelenkkompartiment und ein zweites durch einen zweiten Gelenkkopf und eine zweite Gelenkpfanne gebildetes Gelenkkompartiment, wobei Kontaktflächen der jeweiligen Gelenkkompartimente in der Hauptfunktionsebene einen Versatz aufweisen.

Aus der deutschen Patentanmeldung DE 39 08 958 A 1 ist bereits ein zum Ersatz menschlicher Gelenke bestimmtes künstliches Gelenk bekannt, welches aus zwei Gelenkteilen mit beweglichen Funktionsflächen besteht. Die Krümmungsverhältnisse der eine kreisförmige Schnittkontur aufweisenden Funktionsflächen sind zueinander konvex-konvex, konvexkonkav oder konkav-konkav und die Gelenkgeometrie ist durch eine Gelenkkette mit zwei Gelenkachsen (dimere Gelenkkette) bestimmt, die durch die Rotationszentren der Funktionsflächen verlaufen. Hierbei sind die Gelenkflächen kugelförmig ausgebildet, so dass eine Gelenkbewegung mit fünf Freiheitsgraden möglich ist.

Durch die EP 07 34 700 A 2 ist auch bereits ein künstliches Gelenk bekannt, bei dem die Gelenkgeometrie der Funktionsflächen zueinander in jeder der beiden Ebenen durch eine Gelenkkette mit zwei Gelenkachsen bestimmt ist, die durch die Rotationszentren der Funktionsflächen mit den Radien der jeweils zugehörigen Schnittkonturen verlaufen, wobei eine femurseitige, also gelenkkopfseitige Verbindung der Mittelpunkte der Gelenkköpfe einem Gestell und eine tibiaseitige, also gelenkpfannenseitige Verbindung der Mittelpunkte der Gelenkpfannen einer Koppel einer die vier Achsen aufweisenden Viergelenkkette entsprechen. Es wird dadurch insbesondere erreicht, dass im Flexionsbereich des Gelenkes zwischen 0 und 30° im Kontakt der Gelenkflächen ein hoher Rollanteil erreicht wird. Dabei wandern die Kontaktflächen bezogen auf die Gelenkpfanne zunächst nach posterior, während bei einer weiteren Flexion eine Gleitbewegung ohne Verlagerung der Kontaktfläche stattfindet. Damit ist ein Herausrollen des Gelenkkopfes aus der Gelenkpfanne nicht möglich.

Der Erfindung liegt die Aufgabe zugrunde, die Eigenschaften des künstlichen Gelenkes, basierend auf den genannten Erkenntnissen, weiter zu verbessern. Dabei soll insbesondere eine für den Patienten deutlich spürbare Verbesserung der Bewegungsabläufe unter Annäherung an den natürlichen Bewegungsablauf realisiert werden.

Diese Aufgabe wird erfindungsgemäß mit einem künstlichen Gelenk gemäß den Merkmalen des Patentanspruchs 1 gelöst. Die Unteransprüche betreffen besonders zweckmäßige Weiterbildungen der Erfindung.

Erfindungsgemäß ist also ein künstliches Gelenk vorgesehen, bei dem die Kontaktflächen der beiden Gelenkkompartimente in Abhängigkeit des Flexionswinkels derart geneigt angeordnet sind, dass die Flächennormalen der Kontaktflächen bei jedem Flexionswinkel einen gemeinsamen Schnittpunkt aufweisen. Die Erfindung geht dabei von der Überlegung aus, dass eine resultierende Kraft, insbesondere ein Drehmoment, infolge der Bodenreaktionskraft bei den verschiedenen Flexionswinkeln in optimaler Weise dadurch vermeidbar ist, dass die Kontaktflächen derart aufeinander abgestimmt sind, dass die Wirklinien der an den Kontaktflächen eingeleiteten Kräfte einander schneiden. Hierzu sind die Kontaktflächen derart geneigt angeordnet, dass sich die Flächennormalen im Raum schneiden. Diese Anordnung der Kontaktflächen führt daher zu einer Selbststabilisierung des Gelenkes bei der Einleitung einer Rotationsbewegung bzw. Torsion um die der Gelenkpfanne zugeordneten Tibia. Darüber hinaus ist das künstliche Gelenk auch geeignet, die Einleitung seitlicher Kräfte auszugleichen und die gewünschte Stabilisierung herbeizuführen. Hierdurch wird für den Patienten eine spürbare Verbesserung des Bewegungsablaufes erreicht. Aufgrund der konvexen bzw. konkaven Formgebungen der Gelenkköpfe bzw. der Gelenkpfannen führt eine Änderung des Flexionswinkels zu einer Änderung der Winkelstellung der dadurch resultierenden Kontaktflächen.

Dabei erweist sich eine Ausführungsform der Erfindung als besonders vorteilhaft, wenn die Kontaktflächen eine zur Gelenkmitte aufsteigende Neigung aufweisen. Hierdurch werden mögliche, von der Oberfläche der Kontaktflächen abgetragene Partikel oder sonstiger Abrieb seitlich ausgetragen und führen dadurch nicht zu einer Funktionsstörung des Gelenkes.

Besonders vorteilhaft ist dabei auch eine Weiterbildung, bei der die Kontaktflächen beiderseits einer die Gelenkpfannen des ersten und des zweiten Gelenkkompartimentes unterteilenden, konvexen Krümmung im Übergangsbereich zu einer konkaven Ausformung angeordnet sind. Hierdurch führt auch die Einleitung von Reaktionskräften in einer von der Hauptfunktionsebene abweichenden Richtung zu einer Veränderung der entsprechenden Neigung der Kontaktflächen und damit zu einer der unerwünschten Auslenkung entgegenwirkenden Rückstellkraft, basierend auf der Bodenreaktionskraft, die zu einer zuverlässigen Stabilisierung des Gelenkes führt.

Dabei hat es sich als besonders praxisnah erwiesen, wenn die Flächennormalen der beiden Kontaktflächen einen Winkel von maximal 40° zu der Wirkrichtung der das Gelenk belastenden, resultierenden Gelenkkraft einschließen. Durch diese Neigung der Kontaktflächen wird im Hinblick auf den Bewegungsablauf einerseits die gewünschte Stabilisierung des Gelenkes, andererseits zugleich auch eine Ausweichbewegung bei insbesondere schlagartig eingeleiteten, äußeren Kräften ermöglicht.

Dabei weisen die Kontaktflächen nach einer weiteren, besonders erfolgversprechenden Ausgestaltung einen jeweils unterschiedlichen Neigungswinkel, bezogen auf die beiden Gelenkkompartimente und/oder den Flexionswinkel auf. Hierdurch wird der erfindungsgemäße Vorteil der Selbststabilisierung auch bei einer Viergelenkanordnung realisierbar, deren vorteilhafte Wirkung dadurch genutzt werden kann.

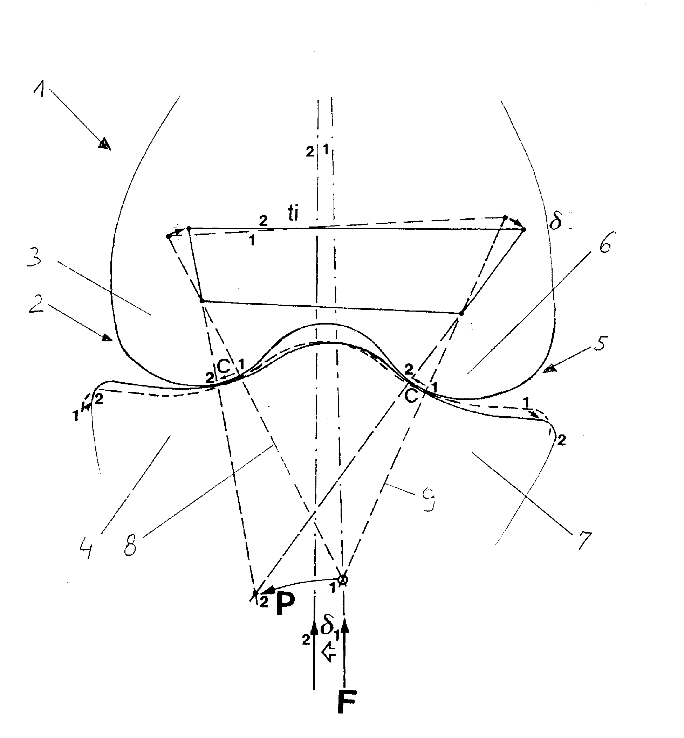

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in einer quer zur Hauptfunktionsebene geschnittenen Prinzipdarstellung ein künstliches Gelenk 1, ein erstes Gelenkkompartiment 2 bildenden ersten Gelenkkopf 3 und eine erste Gelenkpfanne 4 und einen ein zweites Gelenkkompartiment 5 bildenden zweiten Gelenkkopf 6 und eine zweite Gelenkpfanne 7.

Zu erkennen ist, dass zwei Kontaktflächen C der Gelenkkompartimente 2, 5 auf den Abhängen der den Gelenkpfannen 4, 7 zugeordneten, tibialen Kontur und auf der den Gelenkköpfen 3, 6 zugeordneten, femoralen Kontur auf den Innenseiten liegen. Eine seitlich eingeleitete Relativbewegung der Gelenkpfannen 4, 7 gegenüber den Gelenkköpfen 3, 6 führt zu einer Verlagerung von den Kontaktflächen C 1 zu den Kontaktflächen C 2 und damit der zugehörigen Flächennormalen 8, 9. Diese Bewegung entspricht einer Rotation der den Gelenkpfannen 4, 7 zugeordneten Tibia mit einem Winkel δ um eine momentane Drehachse P, die mit der Bewegung gleichzeitig aus der Position P1 nach P2 wandert. Diese Bewegung wird durch ein Viergelenk modelliert, das sich aus den Krümmungsmittelpunkten der Konturen ableitet. Die Strecke ti ist der Abstand der tibialen Mittelpunkte und ist ortsfest im bewegten Koordinatensystem der Tibia, wobei die Strecke ti ebenfalls um den Winkel δ schwenkt. Die Kraftwirkungslinie der resultierenden Gelenkkraft F, die sich insbesondere aus Muskel- und Gewichtskräften zusammensetzt, schwenkt ebenfalls um den Winkel δ, weil auch die tibialen Ansatzpunkte der Muskelkräfte um P mit dem Winkel δ drehen. Da bei geeigneter Neigung der Gelenkflächen in den Kontaktflächen C2 bzw. die momentane Drehachse P weiter nach außen wandert, als die Kraftwirkungslinie, erzeugt in der Position P2 die Gelenkkraft F bezüglich P ein Drehmoment, das der Rotation um den Winkel δ entgegenwirkt. Durch die Anordnung der Kontaktflächen C auf den Abhängen der Gelenkflächen wird bei geeigneten Neigungen derselben ein mechanisch stabiles Gleichgewicht bezüglich Ab-Adduktion erreicht. Eine entsprechende Stabilisierung wird auch für axiale Rotation der Tibia um ihre Längsachse erreicht. Durch die Neigungen der beiden Kontaktflächen C in den Gelenkkontakten der Gelenkflächen, entsprechend eines jeden möglichen Flexionswinkels, führt die axiale Rotation zu einer Distanzierung, zu einem "Auseinanderschrauben" von Femur und Tibia. Durch die kompressive Gelenkkraft F jedoch wird diesem Auseinanderschrauben entgegengewirkt und die Ausgangslage mechanisch stabilisiert. Da wegen des Funktionsviergelenkes jedem Flexionswinkel je eine bestimmte Position der medialen bzw. lateralen Kontaktflächen C 1 und C 2 in der Hauptfunktionsebene vorgegeben ist, sind die Neigungen der Tangentenflächen in diesen Kontaktpositionen konstruktiv so optimiert, dass der Effekt der Selbststabilisierung in jeder Gelenkposition eintritt.

## Patentansprüche

1. Ein insbesondere als Endoprothese für das menschliche Kniegelenk bestimmtes künstliches Gelenk (1), umfassend ein erstes durch einen ersten Gelenkkopf (3) und eine erste Gelenkpfanne (4) gebildetes Gelenkkompartiment (2) und ein zweites durch einen zweiten Gelenkkopf (6) und eine zweite Gelenkpfanne (7) gebildetes Gelenkkompartiment (5), wobei zwei Kontaktflächen (C) der jeweiligen Gelenkkompartimente (2, 5) in der Hauptfunktionsebene einen Versatz aufweisen, **dadurch gekennzeichnet, dass** die Kontaktflächen (C) der beiden Gelenkkompartimente (2, 5) in Abhängigkeit des Flexionswinkels derart geneigt angeordnet sind, **dass** die Flächennormalen (8, 9) der Kontaktflächen (C) bei jedem Flexionswinkel einen gemeinsamen Schnittpunkt aufweisen.

2. Gelenk (1) nach Anspruch 1, **dadurch gekennzeichnet, dass** die Kontaktflächen (C) eine zur Gelenkmitte aufsteigende Neigung aufweisen.

3. Gelenk (1) nach den Ansprüchen 1 oder 2, **dadurch gekennzeichnet, dass** die Kontaktflächen (C) beiderseits einer die Gelenkpfannen (4, 7) des ersten und des zweiten Gelenkkompartimentes (2, 5) unterteilenden, konvexen Krümmung im Übergangsbereich zu einer konkaven Ausformung angeordnet sind.

4. Gelenk (1) nach zumindest einem der vorhergehenden Ansprüche, **dadurch gekennzeichnet, dass** die Flächennormalen (8, 9) der beiden Kontaktflächen (C) einen Winkel von maximal 40° zu der Wirkrichtung der das Gelenk (1) belastenden, resultierenden Gelenkkraft (F) einschließen.

5. Gelenk (1) nach zumindest einem der vorhergehenden Ansprüche, **dadurch gekennzeichnet, dass** die Kontaktflächen (C) einen jeweils unterschiedlichen Neigungswinkel aufweisen.
